# EUROPEAN PATENT SPECIFICATION

(11) **EP 2 856 984 B1**
(45) Date of publication and mention of the grant of the patent: **23.03.2016**
(21) Application number: 14191095.0
(22) Date of filing: 02.05.2011
(51) Int. Cl.: A61F 5/445

(54) **Disposable ostomy cap**
Einweg-Ostomieabdeckung
Bouchon de stomie jetable

(30) Priority: 02.05.2010 US 201061330359 P; 14.07.2010 WO PCT/IL2010/000565; 10.01.2011 US 201161431084 P
(43) Date of publication of application: 08.04.2015
(62) Divisional of application: 11723674.5
(73) Proprietor: B.Braun Medical SAS, 92100 Boulogne-Billancourt (FR)
(72) Inventor: Hanuka, David, 30095 Ramat-Yishai (IL); Or, Meir, 20196 Doar-Na Misgav (IL)
(74) Representative: Mahler, Peter

(56) References cited:
- WO-A1-02/058603
- WO-A2-2009/083183
- US-A- 6 050 982

## Description

### FIELD OF THE INVENTION

The present invention, in some embodiments thereof, relates to ostomy ports and, more particularly, but not exclusively, to a disposable ostomy cap for use with ostomy ports and/or other parts, such as an ostomy bag or a whole port.

### BACKGROUND OF THE INVENTION

Following a stoma operation, an ostomy port may be inserted through the stoma for controlling discharge of waste content through the stoma. An ostomy bag may be attached to a proximal opening in the port into which the waste content is discharged. The bag may require replacing several times a day according to an amount of waste content accumulating in the bag.

As an alternative to having a bag attached at all times to the port, some ports are configured to be sealed by a cap at the proximal opening. In these ports, the waste content does not flow out the proximal opening until the cap is removed. Rather, the waste content accumulates inside the port until released by a user. Generally, the user of the ostomy port first attaches an ostomy bag or some other waste collection element to the port and only then removes the cap to allow waste content discharge into the bag.

In U.S. Patent No. 6,033,390 von Dyck describes "a continent ostomy port device has a face plate defining an aperture alignable with the opening of a stoma in the user's body and a closure adjacent to the aperture is adapted to permit covering and uncovering of the aperture in the face plate. A catheter extends from one side of the face plate proximally, and one end of the catheter is disposed within the ostomy site when the port device is in use. The catheter has continuous exterior and interior side walls, the latter defining a major lumen and is sized and shaped for non-surgical insertion through a stoma to a sufficient distance that the presence of the catheter within the stoma provides a barrier which reduces the incidence of prolapse, without the use of extraneous, externally applied materials or additional surgery. A removable cartridge fits snugly and slideably within the major lumen of the catheter of the device so as to prevent inadvertent escape of body waste material from the stoma when the cartridge is in place, without use of an ostomy bag, and to clean the interior side wall of the catheter as the cartridge is pressed into the major lumen. An anti-reflux valve is activated to prevent escape of body waste and deactivated for passage of fluid. Retaining structure is connected to the catheter, and is non-surgically, snugly fittable into the stoma, to cause the port device to be self-retaining in a normal use position within a stoma, without surgery or fixation materials."

In U.S. Patent No. 6,050,982 to Wheeler, representing the closest prior art, is described "a concealed colostomy apparatus comprising a sleeve insertible into the bowel via a discharge opening thereof for retention therein, the sleeve then having a discharge end; a cap removably interfitting the discharge end of the sleeve; and a flexible pouch received in collapsed position into the sleeve to in turn receive feces from the bowel, the cap being removable to allow distending of the pouch outside the sleeve and continued filling of fecal matter into the pouch.

Additional background art includes U.S. Patent Nos. 4,121,589; 4,338,937; 4,634,421; 4,721,508; 4,381,765; and 4,662,890.

### SUMMARY OF THE INVENTION

There is provided in accordance with an exemplary embodiment of the invention, a disposable cap for sealing a proximal opening in an ostomy port, including a coupler adapted to couple to an ostomy port and comprising a collapsed ostomy bag. In an exemplary embodiment of the invention, the bag is housed in a body of the cap. Optionally or alternatively, the cap is wider at a proximal side thereof than at a distal side thereof. In an exemplary embodiment of the invention, the cap is configured to extend less than 1 cm proximally from the port. In an exemplary embodiment of the invention, the cap is configured to fit into the port.

In an exemplary embodiment of the invention, the cap is configured to interlock with an interlocking mechanism of the port.

In an exemplary embodiment of the invention, the bag is configured to deploy when a pressure in the port is above a threshold.

In an exemplary embodiment of the invention, the bag includes a pullable element for manual deployment.

In an exemplary embodiment of the invention, the bag includes a deployment prevention element.

In an exemplary embodiment of the invention, the cap includes a pressure indicator.

In an exemplary embodiment of the invention, the cap includes a sensing circuitry.

In an exemplary embodiment of the invention, the cap includes a foil seal.

In an exemplary embodiment of the invention, the cap includes a separately openable and closable cap-covering.

In an exemplary embodiment of the invention, the cap has a sleeve shaped body and wherein the cap-covering is configured to fit on two opposite sides of the body.

In an exemplary embodiment of the invention, the cap includes a separately openable cap-covering.

In an exemplary embodiment of the invention, the bag is elastic.

In an exemplary embodiment of the invention, the bag is integrally formed with the cap.

In an exemplary embodiment of the invention, the bag is permanently attached to the cap.

In an exemplary embodiment of the invention, the cap includes a closure for sealing the bag after filling thereof.

In an exemplary embodiment of the invention, the cap includes an adhesive attachment element for attaching the bag after deployment directly to a body of the patient.

In an exemplary embodiment of the invention, the bag has a volume when collapsed of less than 10 cc and when full of at least 300 cc.

There is provided in accordance with an exemplary embodiment of the invention, a method of using an ostomy port, comprising:
(a) determining that waste needs to be evacuated from the port;
(b) deploying a bag from the port without opening the port to outside of the bag;
(c) removing the bag with waste inside; and
(d) sealing the port.

In an exemplary embodiment of the invention, deploying a bag comprises opening a cover of a cap of the port.

In an exemplary embodiment of the invention, removing the bag comprises sealing the bag. Optionally, sealing the bag comprises sealing with a cover of the cap. Optionally or alternatively, sealing the bag comprises sealing by narrowing a portion of the bag. Optionally or alternatively, sealing the port comprises sealing with one or both of a collapsed bag and a cap. Alternatively, sealing the port comprises sealing without a bag.

There is provided in accordance with an exemplary embodiment of the invention, a disposable ostomy port comprising:
a tube insertable through a stoma into in an intestinal portion for conducting a flow of waste content from the intestinal portion; and
a retention balloon for affixing the tube to the stoma and configured for contact with tissue;
wherein the tube and the retention balloon are formed as a single component. Optionally, the port comprises a stomal cover formed as part of the single component.

There is provided in accordance with an exemplary embodiment of the invention, a disposable ostomy port including a waste tube and a lumen distally connected to the balloon for inflating the balloon therethrough with an inflation fluid and including an inflation valve.

In an exemplary embodiment of the invention, the tube includes a sealed proximal end.

In an exemplary embodiment of the invention, the port comprises a gas filtering mechanism.

In an exemplary embodiment of the invention, the tube includes an ostomy bag attached to a proximal end thereof.

In an exemplary embodiment of the invention, the tube has a proximal opening configured to attachment of an ostomy bag thereto.

There is provided in accordance with an exemplary embodiment of the invention, a sensing cap configured for attachment to an ostomy port opening and comprising at least one distensible element, which distends in response to a pressure in the port.

There is provided in accordance with an exemplary embodiment of the invention, an ostomy port adapted for insertion into the body and including a non-detachable waste bag.

There is provided in accordance with an exemplary embodiment of the invention, an ostomy bag coupled with a closure element for sealing thereof. Optionally, the bag has a rim for mounting on an ostomy port and wherein the closure element comprises a cap that fits on the rim. Optionally, the cap is configured to fit on either side of the rim. Optionally or alternatively, the closure element comprises a tie.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Figures 1A and 1B schematically illustrate a perspective view of an exemplary disposable cap and an exploded perspective view of the cap, respectively, according to an embodiment of the present invention;
Figure 2 schematically illustrates a sectional view of the cap, according to an embodiment of the present invention;
Figures 3A and 3B schematically illustrate the cap attached to an ostomy port having a stomal cover, in a waste continence mode and in a waste collection mode, respectively, according to some embodiments of the present invention;
Figure 4 schematically illustrates an exemplary disposable cap including a pressure sensing cover, according to some embodiments of the present invention;
Figure 5 schematically illustrates an exemplary disposable cap including an ostomy bag housed in a bag cavity formed in a capsule between a proximal opening and a cover, according to some embodiments of the present invention;
Figure 6 schematically illustrates an exemplary disposable cap including capsule covered by a removable film lid, according to some embodiments of the present invention;
Figures 7A and 7B schematically illustrate an exemplary disposable cap including a reusable ostomy bag deployed from a capsule, according to some embodiments of the present invention;
Figures 8A and 8B schematically illustrate a method of closing an exemplary ostomy bag in a disposable cap following collection of waste content from an ostomy port, according to some embodiments of the present invention;
Figures 9A to 9C schematically illustrate an exemplary disposable cap with a supported deployed ostomy bag, according to some embodiments of the present invention;
Figure 10 schematically illustrates an exemplary ostomy port including an elastic stomal cover, an inflatable balloon, and an elastic tube all integrally formed as a single component suitable for one-time use, according to some embodiments of the present invention;
Figure 11 schematically illustrates an exemplary ostomy port for a one-time use with a built-in ostomy bag for collecting waste content, according to some embodiments of the present invention;
Figure 12 schematically illustrates an ostomy port including a removable cap for removing waste content, according to some embodiments of the present invention;
Figures 13A - 13C schematically illustrate an exemplary ostomy port including an attachment mechanism for attaching an elastic collection bag, according to some embodiments of the present invention;
Figures 14A - 14D schematically illustrate an ostomy port including an attachment mechanism for attaching a collection bag used in domestic applications (for example, a sandwich bag, small garbage bags, and the like) to a proximal end of an elastic tube, according to some embodiments of the present invention;
Figure 15 illustrates a flow chart of a method for using a disposable cap with an ostomy port, according to an embodiment of the present invention;
Figure 16 schematically illustrates an attachment mechanism for attaching a disposable cap with an ostomy bag inside a capsule into an ostomy port, according to some embodiments of the present invention;
Figure 17 schematically illustrates a quick release mechanism for removing a cover from a disposable cap for use with an ostomy port, according to some embodiments of the present invention; and
Figures 18A - 18H illustrate an exemplary method of furling an ostomy bag into a disposable cap and placing a cover on the cap, according to some embodiments of the present invention.

### DESCRIPTION OF SPECIFIC EMBODIMENTS OF THE INVENTION

The present invention, in some embodiments thereof, relates to ostomy ports and, more particularly, but not exclusively, to a disposable ostomy cap for use with ostomy ports and/or other parts, such as an ostomy bag or a whole port.

Reference hereinafter to an ostomy port or a stomal cover may include any of the embodiments described in any one of the applications from which this disclosure is claiming benefit and referenced in the above section Related Application.

An aspect of some embodiments of the present invention relates to a disposable cap for sealing a proximal opening of an ostomy port. The cap includes an ostomy bag which is deployable for collecting waste content flowing through the ostomy port and out the proximal opening.

As used hereinafter, distal refers to a direction away from the proximal opening and towards an interior of the abdominal cavity while proximal refers to a direction away from the abdominal cavity towards the proximal opening.

In some exemplary embodiments, the ostomy bag includes a collection volume for waste content in a range of 200 - 1000 ml, for example, 400 ml, 500 ml, 600 ml, 700 ml. Optionally, for irrigation purposes, the ostomy bag includes a volume ranging from 1000 - 1500 ml, for example, 1100 ml, 1250 ml, 1350 ml, 1450 ml. Additionally, a film thickness of the ostomy bag ranges between 50 - 200 µm, for example, 60 µm, 80 µm, 100 µm, 120 µm

In some exemplary embodiments, the cap acts as a capsule for housing the bag. Optionally, the cap may include a coupling section for coupling to the ostomy port (may be referred to hereinafter also as a " coupler"). The capsule material may include plastic, hardened rubber, and/or any other waste content resistant material suitable for housing the bag and for being inserted into a proximal opening. An internal cavity in the capsule accommodating the bag may, for example, have a volume ranging between 3000 - 15000 mm³, for example, 5000 mm³, 6000 mm³, 7000 mm³. Optionally, the edge of an opening to the bag is adhered to the capsule by welding. Alternatively, the bag is adhered to the capsule by bonding or by any other method known in the art and suitable for adhering the bag material with the capsule material. Optionally, the capsule is conically shaped for providing for a greater volume of space into which the bag may be folded or furled due to an increasing cross-sectional area in the capsule in the proximal direction. Additionally or alternatively, the conical capsule allows for easier insertion of the cap into the proximal opening. This may reduce bulging of the cap in the proximal direction, making it less noticeable to the user or other people and/or easier for insertion into the proximal opening of an ostomy port. Additionally or alternatively, the conical capsule reduces the possibility of the bag bulging out in a distal direction from a distal opening of the capsule when furled inside the capsule. Optionally, the proximal end in the ostomy port is conically shaped for accommodating the cap.

In some exemplary embodiments, the cap includes a plastic cap cover. Alternatively, the cap does not have a cover on either a proximal end or a distal end. A user wishing to discharge waste content into the ostomy bag removes the cover and pulls out the bag. Alternatively, the cap cover includes an elastomeric material. Alternatively, the cap cover includes a flexible film. Alternatively, the cover is a lid which may be removed by stripping or peeling off the cap. Alternatively, the cap cover includes any other material suitable to cover a proximal opening in the capsule housing the folded and/or furled bag. Alternatively, the bag is held in an internal cavity of the capsule by a friction force, and may be manually pulled out by a user. Alternatively, the bag is held in an internal cavity of the capsule by a mechanical fixation element (e.g. an R-clip) that may be manually pulled out by a user.

In some exemplary embodiments, the cap is attached to the ostomy port using a twist-and-lock mechanism. Alternatively, the attachment includes a snap-lock mechanism. Alternatively, the attachment includes threading the cap into the proximal opening of the ostomy port. Alternatively, the cap may be integral to the ostomy port or to an insert to the port. Alternatively, other methods known in the art suitable for fastening the cap to the proximal opening may be used.

In some exemplary embodiments, the cap includes a sensing mechanism for communicating to the user of a need for evacuation by sensing an increase in intestinal pressure. Optionally, the cap includes a cover equipped with a flexible portion adapted to protrude (bulge) outwards in the proximal direction when exposed to axial pressure exerted by waste content or flatus. Alternatively, the bag may additionally serve as the cover. The protrusion distance in response to an axial pressure of 50 mmHg may range from 2 - 30 mm, for example, 3 mm, 5 mm, 7 mm, 10 mm. Optionally, the flexible portion is concave shaped. In some embodiments, the material of the sensing cover may be an elastomer such as, for example, silicone rubber, and may be of a durometer ranging between 5 - 10 Shore A, for example, 40 shore A, 60 shore A, 70 shore A, 75 shore A. A thickness may range between 0.2 - 2 mm, for example 0.75 mm, 1 mm, 1.25 mm.

In some exemplary embodiments, the sensing mechanism includes logic implemented in the port for sensing the increase in intestinal pressure. Alternatively, the logic is implemented in the cap. Optionally, upon reaching a predetermined value, a visual and/or audible and/or sensible warning is generated for notifying the user of the increase pressure. The indication may be a mechanically activated protruding element which may be seen by the user, or an electrical or electromechanical indication such as, for example, a light, a vibration, a sound and/or a wireless signal (e.g., Bluetooth).

In some exemplary embodiments, the port includes a safety mechanism for releasing gas and/or waste content. The safety mechanism may eject the cover or a section of the cap when the axial pressure reaches the predetermined value. Optionally, the safety mechanism includes a pressure-sensitive mechanism. Additionally or alternatively, the safety mechanism includes logic circuitry and/or mechanical logic for determining when to release the cover. Optionally, the safety mechanism includes electro-mechanical components. Optionally, the bag is pushed out of the capsule by an axial pressure ranging between 50 mmHg - 100 mmHg, for example, 65 mmHg, 75 mmHg, 90 mmHg, 95 mmHg. Once the waste content has been expelled or the colonic pressure has decreased below the predetermined value, the cover may be replaced onto the cap. Optionally, to exclude false alarms due to temporary colonic pressure pulses, the cover is released when a colonic pressure inside the ostomy port is equal to or greater than, for example, 60 mmHg for a period of time greater than 5 seconds, greater than 15 seconds, greater than 30 seconds, greater than 60 seconds, greater than 90 seconds. Optionally, the cover is released when the colonic pressure is greater than or equal to 80 mmHg, greater than or equal to 100 mmHg, greater than or equal to 150 mmHg, greater than or equal to 200 mmHg.

In some exemplary embodiments, a pressure sensor (not shown) is assembled in an interior of the ostomy port, for example on an internal wall of the ostomy port, and a control unit is assembled at a portion of the ostomy port externally to the user's body, for example on the stomal cover. The control unit receives pressure signals from said pressure sensor, and is programmed with a logic algorithm for selectively opening a gas release valve upon fulfillment of predetermined conditions, for example any of the following conditions:
a. Internal pressure is greater than60 mmHg for more than 1 min;
b. Internal pressure is greater than 100 mmHg for more than 10 sec;
c. Internal pressure is greater than 150 mmHg, immediate release.

Optionally, the ostomy port is equipped with an indication mechanism, for example visual, audible or vibrational alarm, to notify the user of an activation of the gas release valve.

Additionally or alternatively, the control unit notifies the user on a need to release gas without automatically activating the gas release valve. Optionally, the gas release valve can be closed either manually by the user or automatically by said control unit when the internal pressure decreases, for example, to no greater than 30 mmHg.

In some exemplary embodiments, a pressure sensor and a control unit as those described above control the opening of a gas release valve and/or deploying of a disposable collection bag, according to a predetermined logic, for example:
a. As internal pressure is greater than 60 mmHg for more than 1 min, open the gas release valve;
b. As internal pressure is greater than 60 mmHg for more than 2 min, deploy the disposable collection bag;
c. As internal pressure is greater than 100 mmHg for more than 10 sec, open the gas release valve;
d. As internal pressure is greater than 100 mmHg for more than 30 sec, deploy the disposable collection bag;
e. As internal pressure is greater than 150 mmHg, , open the gas release valve immediately;
f. As internal pressure is greater than 150 mmHg, deploy the disposable collection bag immediately;

Optionally, an alert may be sent over wireless to the user warning of a high pressure condition, for example, through Bluetooth to a receiver carried or worn by the user. Optionally, the user may be able to program the control for setting pressure levels and duration, type of alarm to activate and duration of the alarm, among other possible control features. The user may additionally control activation of a blocking element inside the port such as, for example, a balloon, a gas release valve, and the like.

In some exemplary embodiments, the cap includes a discharge content indicator and/or a safety release valve in case of excessive waste content inside the ostomy port. Additionally or alternatively, the cap includes a gas filter for filtering gas, including flatus, from inside the ostomy port and for releasing to the ambient.

In some exemplary embodiments, the ostomy bag is unfurled by the pushing of the waste content. Optionally, a portion of the bag is left unfurled for pulling by the user. Additionally or alternatively, a strap or cord is attached to the bag, either at a proximal or distal end, for the user to pull on for unfurling the bag.

In some exemplary embodiments, the ostomy bag includes a strand for closing the bag prior to disposal. Optionally, the strand is adapted to tie the bag along an upper portion of the bag. Optionally, the bag may be closed by a cap for sealing the opening to the bag. Additionally or alternatively, the cap is placed on the upper portion of the bag. Optionally, the bag is closed by means of a clasp, a string, a tie, or any other means known in the art for closing the bag.

In some exemplary embodiments, the ostomy bag is open ended for allowing flushing of the waste content inside the bag. A sleeve may be connected to the open end for directing the waste content into a toilet. Flushing may be performed while the cap is removed from the proximal opening of an ostomy port. Additionally or alternatively, flushing may be performed while the cap is connected to the proximal opening by injecting water through an irrigation port on the ostomy port. Optionally, the bag is reusable by closing the open end of the bag and/or the sleeve.

In some exemplary embodiments, the ostomy bag is supported against the body of the user for reducing pressure on the internal abdominal wall and/or on the intestine due to the weight of a full or partially full bag pulling on the ostomy port. The use of a support may be suitable for applications involving an ileostomy or a urostomy where the ostomy bag is deployed at all times for continuously collecting waste content. Additionally or alternatively, the use of a support may be suitable for applications where accumulating of waste matter inside the intestine is undesirable and the ostomy bag is hence deployed at all times. Additionally or alternatively, the use of a support may be suitable for bowel irrigation purposes. A tab may be included on the bag, for example on a top section, for adhering the bag to the user's abdomen for providing weight support. The tab may apply a tensile force for retaining the bag as it grows heavier with the accumulation of waste content. Additionally or alternatively, a tab may be included in a bottom section of the bag for adhering to a body portion of the user for providing additional support. Additionally or alternatively, a belt may be used for securing the bag against the user's waist. Additionally or alternatively, the stomal cover may be adhered to the user's abdomen for supporting the bag.

An aspect of some embodiments of the present invention relates to a disposable ostomy port including one or more features in a single component. Optionally, the ostomy port is for a one-time use. This may allow for relatively inexpensive and simple production of the port. The ostomy port includes a tube which connects at a distal end to an intestinal portion and a retention balloon for retaining the tube in a stoma, the balloon and the tube produced together from a same material. The material may be an elastic and flexible material such as, for example, silicone rubber, and may be of a durometer ranging from 10 - 80 Shore A, for example, 20 Shore A, 30 Shore A, 40 Shore A. Optionally, the port is produced by attaching a free end of the balloon to an external surface of the tube during assembly. In some embodiments, the balloon may have an inflation volume ranging from 25 - 100 ml for colostomy, and from 15 - 50 ml for ileostomy. A wall thickness of the balloon may range from 0.2 mm - 1.5mm, for example, 0.3 mm, 0.5 mm, 0.8 mm. The tube may have an internal diameter ranging from 15 - 35 mm for colostomy and from 10 - 25 mm for ileostomy and urostomy. A wall thickness of the tube may range from 0.3 mm - 2 mm, for example, 0.8 mm, 1 mm, 1.2 mm, 1.5 mm, 1.8 mm. A length of the tube varies with an abdominal wall thickness and may range from 1 cm - 15 cm, depending on the degree of slenderness of the user.

In some exemplary embodiment, the port includes a stomal cover. Optionally, the port includes an inflation lumen distally connected to the balloon for introducing an inflation fluid into the balloon. The inflation lumen may have an internal diameter ranging from 0.5 - 3 mm, for example, 1.5 mm, 2 mm, 2.5 mm. Additionally, the inflation lumen is proximally connected to an inflation valve through which the inflation fluid is introduced into the lumen. Optionally, disposable ostomy port includes a gas filtering mechanism.

In some exemplary embodiments, the tube includes a sealed proximal end so that waste content flowing through the tube is retained inside the tube. Alternatively, an ostomy bag attached to the proximal end is formed as a single component with the port. Optionally, the bag is furled up inside the proximal opening of the tube and is deployed for collecting waste content flowing through the tube. The ostomy bag may include a collection volume for waste content in a range of 200 - 1000 ml, for example, 400 ml, 500 ml, 600 ml, 700 ml. Additionally, a bag wall thickness may range form 0.1 mm - 1 mm, for example, 0.3 mm, 0.5 mm, 0.7 mm. Optionally, the ostomy bag is coated with a low permeability material for reducing its permeability, for example, parylene. Additionally or alternatively, a cover is attached to the proximal opening. The cover may be of a rigid material, a semi-rigid material, or a flexible material, and may include a thickness ranging from 0.5 mm - 3 mm, for example, 1 mm, 2 mm, 2.5 mm. Optionally, the cover may be of plastic or a high durometer elastomer, for example at least 70 Shore A. Optionally, the cover is a disposable cap as previously described.

In some exemplary embodiments, an attachment element is formed as a single component together with the tube. Alternatively, the attachment element is formed as a separate component from the tube. The attachment element may serve for attaching the cover to the proximal opening. Additionally or alternatively, the attachment element may serve for attaching an ostomy bag to the proximal opening. The attachment element may be disposable. Alternatively, the attachment element is reusable. The attachment mechanism may be of a rigid material or a semi-rigid plastic or a high durometer elastomer, for example at least 70 Shore A. The elastic collection bag may include a collection volume for waste content in a range of 200 - 1000 ml, for example, 400 ml, 500 ml, 600 ml, 700 ml.

In some exemplary embodiments, an elastic waste content bag is attachable to the proximal opening. The elastic waste content bag may include a stretchable elastomer such as, for example, silicone rubber or latex. The disposable ostomy port includes a rim over which an elastic opening of the bag is stretched, tightedly fitting the bag onto the port. Optionally, the port includes a locking mechanism for securing the elastic opening onto the rim. The locking mechanism may be of a rigid material or a semi-rigid plastic or a high durometer elastomer, for example at least 70 Shore A. The elastic collection bag may include a collection volume for waste content in a range of 200 - 1000 ml, for example, 400 ml, 500 ml, 600 ml, 700 ml.

In some exemplary embodiments, the port is adapted to be fitted with a domestic bag for use as a waste content bag (ostomy bag), for example, a sandwich bag, a small garbage bag, and the like. The port includes a rim around the proximal opening onto which the edge of the bag's opening is fitted. A securing element and a latching element are coupled onto the rim and over the edges of the bag's opening for securing the bag. The securing element and the latching element may be of a rigid material or a semi-rigid plastic or a high durometer elastomer, for example at least 70 Shore A. The securing element and the latching element may be configured for holding a bag of thickness of 50 µm with a pulling force of for example up to 1 kg, when engaged onto one another.

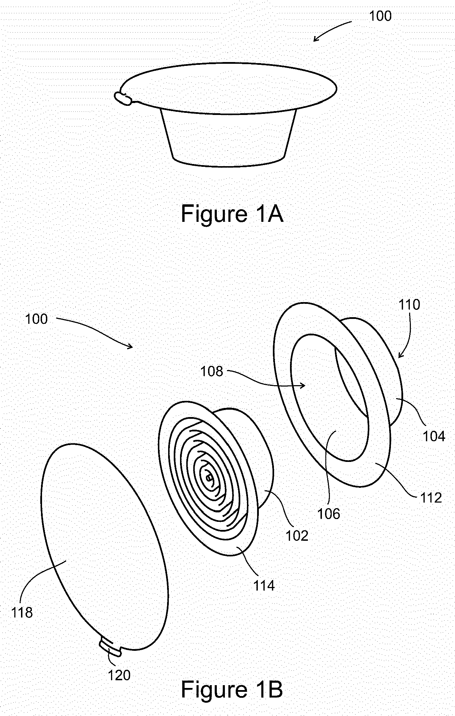

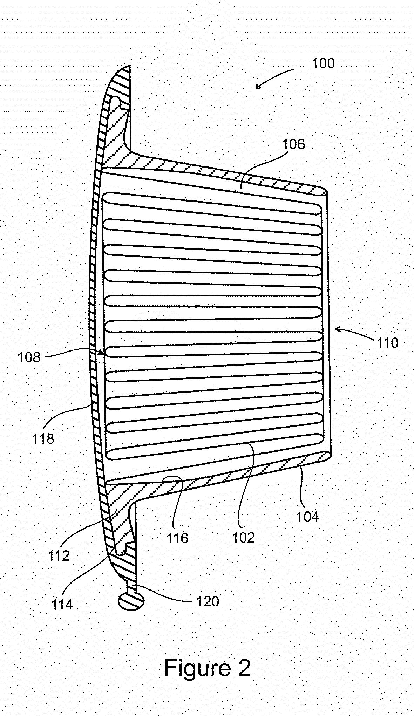

Reference is now made to Figures 1A and 1B which schematically illustrate a perspective view of an exemplary disposable cap **100** and an exploded perspective view of the cap, respectively, according to an embodiment of the present invention. Reference is also made to Figure 2 which schematically illustrates a sectional view of cap **100,** according to an embodiment of the present invention. Disposable cap **100** includes an ostomy bag **102** and a capsule **104** having an interior cavity **106** for housing the bag.

Capsule **104** includes a proximal opening **108** through which bag **102** is optionally inserted into cavity **106** during assembly of cap **100,** and through which the bag is deployed for collecting waste content from an ostomy port. Optionally, bag **102** is folded inside cavity **106.**

Capsule **104** additionally includes a distal opening **110** which fits through, or onto, a proximal opening of an ostomy port, and through which waste content flows from the port into bag **102.** Optionally, capsule **104** is conically shaped with proximal opening **108** having a larger cross sectional area than distal opening **110.** Alternatively, capsule **104** is cylindrically shaped. A rim **112** peripherally bounds proximal opening **108** and is an attachment surface for adhering an edge **114** to an opening into ostomy bag **102.** Alternatively, edge **114** is adhered to an inner wall **116** of cavity **106.**

Cap **100** includes a cover **118** for sealing proximal opening **108** with bag **102** inside cavity **106.** Sealing of proximal opening **108** substantially prevents leakage of waste content through cap **100.** Optionally, cover **118** includes a tab **120** for allowing a user to pull the cover off capsule **104.** Optionally, cover **118** is non-replaceable and cannot be fitted onto capsule **104** following removal. Alternatively, cover **118** is replaceable. Cover **118** may be fitted onto capsule **104** using a snap-lock fastening mechanism, a twist-and-lock fastening mechanism, or any other type of mechanism known in the art and suitable for preventing leakage of waste content from proximal opening **108.**

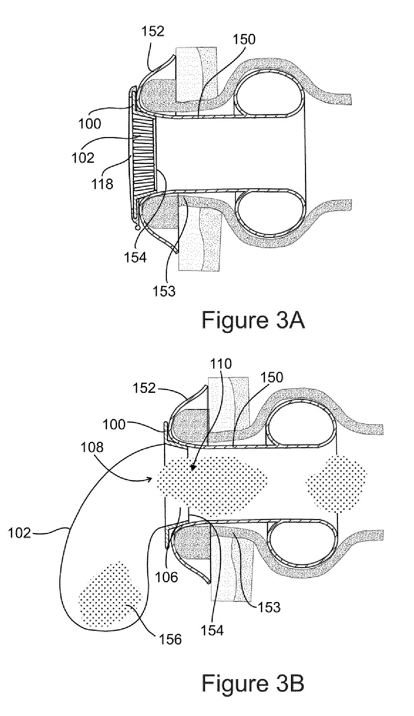

Reference is now made to Figures 3A and 3B which schematically illustrate cap **100** attached to an ostomy port **150** having a stomal cover **152,** in a waste continence mode and in a waste collection mode, respectively, according to some embodiments of the present invention. Ostomy port **150** is inserted in a stoma **153.**

In the waste continence mode, cap **100** is inserted into a proximal opening **154** in ostomy port **150,** sealing against possible leakage of waste content from the ostomy port through the opening. Optionally, cap **100** is attached to stomal cover **152** by a snap-lock mechanism. Alternatively, other fastening mechanisms may be used, for example, a twist-and-lock mechanism, threaded fasteners, or other mechanism known in the art suitable for fastening cap **100** to ostomy port **150** while preventing leakage from proximal opening **154.**

In the waste collection mode, bag **102** is deployed through proximal opening **108** in cap **100.** Waste content **156** flows from ostomy port **150** through distal opening **110** in cap **100** into cavity **106** and therefrom through proximal opening **108** into bag **102.** Optionally, the user removed cover **118** and pulled bag **102** through proximal opening **108** for deploying the bag. Alternatively, bag **102** is pushed out by axial pressure exerted on the bag by waste content **156.** Additionally or alternatively, cover **118** was automatically ejected by the pressure of bag **102** pushing on the cover and/or was released by a pressure sensing mechanism in cap **100.**

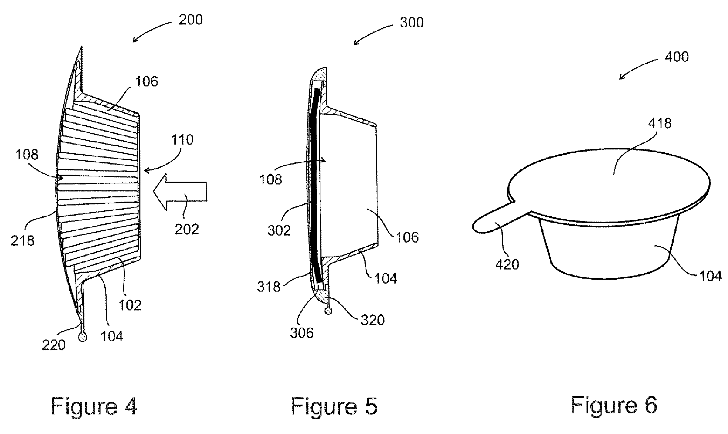

Reference is now made to Figure 4 which schematically illustrates an exemplary disposable cap **200** including a pressure sensing cover **218,** according to some embodiments of the present invention. Cap **200** includes bag **102** inside cavity **106** of capsule **104.**

Pressure sensing cover **218** is made of a stretchable, flexible material and sealingly fits onto capsule **104** over proximal opening **108.** Pressure sensing cover **218** is adapted to protrude in a proximal direction when an axial pressure **202** is applied by waste content and/or flatus through distal opening **110** in the direction of proximal opening **108.** Optionally, bag **102** is pushed in the proximal direction by axial pressure **202** and pushes on cover **218.** Optionally, protruding cover **218** is indicative of a need for the user to evacuate and/or to release flatus. Optionally, the user removes cover **218** by pulling on a tab **220** for releasing bag **102.** Additionally or alternatively, the user releases flatus using a gas release mechanism exsiting in the ostomy port.

Reference is now made to Figure 5 which schematically illustrates an exemplary disposable cap **300** including an ostomy bag **302** housed in a bag cavity **306** formed in capsule **104** between proximal opening **108** and a cover **318,** according to some embodiments of the present invention. Bag cavity **306** is externally located to cavity **106** and is dimensioned for accommodating ostomy bag **302** folded inside. Optionally, the user removes cover **318** by pulling on a tab **320** for releasing bag **102.**

Reference is now made to Figure 6 which schematically illustrates an exemplary disposable cap **400** including capsule **104** covered by a non-reusable film lid **418,** according to some embodiments of the present invention. Film lid **418** may include a plastic or flexible metal. Additionally or alternatively, film lid **418** includes a flexible metal coated with a plastic material suitable for welding onto a surface of capsule **104** (e.g. polyethylene). Optionally, the user removes film lid **418** by pulling on a tab **420.**

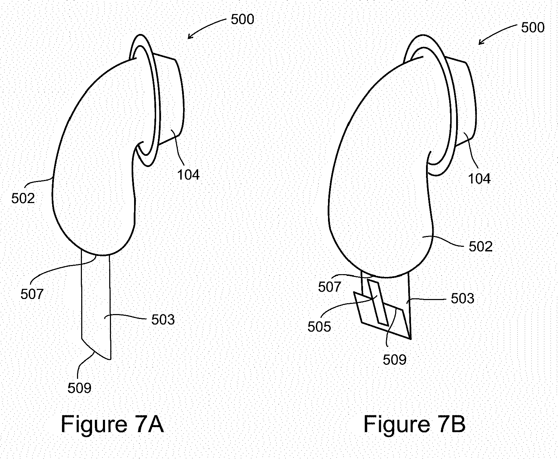

Reference is now made to Figures 7A and 7B which schematically illustrate an exemplary disposable cap **500** including a reusable ostomy bag **502** deployed from a capsule **104,** according to some embodiments of the present invention. Bag **502** includes an opening **507** on a bottom portion of the bag connecting to a sleeve **503.**

Sleeve **503** is used for flushing out waste content inside bag **502** for reusing the bag. Following evacuation, the user may approximate a sleeve opening **509** at the end of sleeve **503** to a toilet or other waste disposal means for discharging the waste content. Optionally, following discharge, the user may wash bag **502** including sleeve **503** for removing any remnants of waste content.

In Figure 7A, cap **500** is shown removed from an ostomy port for discharging the waste content. Alternatively, waste content maybe discharged from bag **502** while cap **500** is attached to the ostomy port.

In Figure 7B, bag **502** is shown with sleeve **503** closed for sealing opening **507** in bag **502** and sleeve opening **509.** Optionally, sleeve **503** is closed when bag **502** is folded inside capsule **104.** Additionally or alternatively, sleeve **503** is closed for reuse following flushing of the waste content. In the closed position, sleeve **503** is folded and/or furled for preventing leakage from the sleeve, and adhered to bag **502.** Optionally, sleeve **503** is adhered to bag **502** using an adhesive strip **505.** Alternatively, other fastening mechanisms may be used, for example, a hook-and-loop fastener.

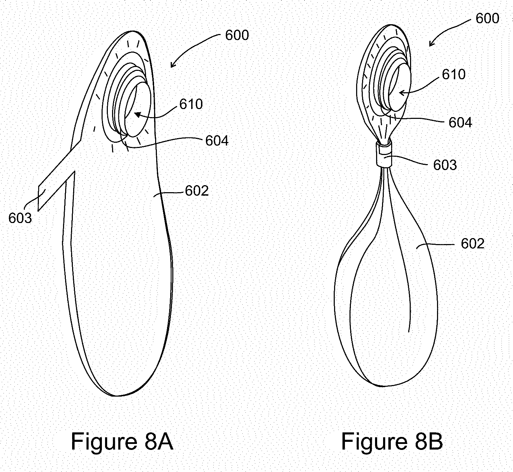

Reference is now made to Figures 8A and 8B which schematically illustrate a method of closing an exemplary ostomy bag **602** in a disposable cap **600** following collection of waste content from an ostomy port, according to some embodiments of the present invention. Waste collection bag **602** includes a strand **603** which is wrapped around a portion of the bag below capsule **604,** closing (clamping) the bag and preventing waste content from reaching the capsule (and spill out distal opening **610**). Alternatively, waste collection bag **602** may include other means of securing the bag below capsule **604** for preventing waste content from reaching the capsule. For example, bag **602** may be clasped below capsule **604** with a clip, or a tie wire, or any other suitable tie means. Optionally, the tie means may be reusable. Alternatively, the tie means are disposed of with bag **602.** Alternatively, a cover may be fitted on distal opening **610** for sealing the opening and preventing waste content from leaking out bag **602.**

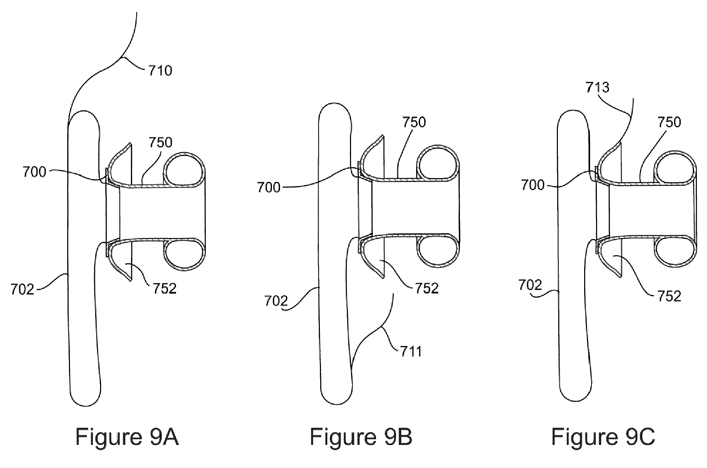

Reference is now made to Figures 9A to 9C which schematically illustrate an exemplary disposable cap **700** with a supported deployed ostomy bag **702,** according to some embodiments of the present invention. Disposable cap **700** is attached to a stomal cover **752** in an ostomy port **750,** bag **702** continuously deployed as may be typical for example for an ileostomy or a urostomy for collecting waste content continuously.

In Figure 9A, a weight of a partially full, or full, ostomy bag **702** is supported by a tab **710** attached to an upper section of the bag and adhered to a body section of the user, for example, to an external abdominal wall. Optionally, tab **710** increases a tensile pulling force on bag **702** as the bag grows heavier with the weight of collected waste content. Additionally or alternatively, ostomy bag **702** is supported by a tab **711** attached to a bottom section of the bag, as shown in Figure 9B. Additionally or alternatively, bag **702** is supported by adhering stomal cover **752** to the external abdominal wall of the user, optionally by a tab **713** attached to the stomal cover.

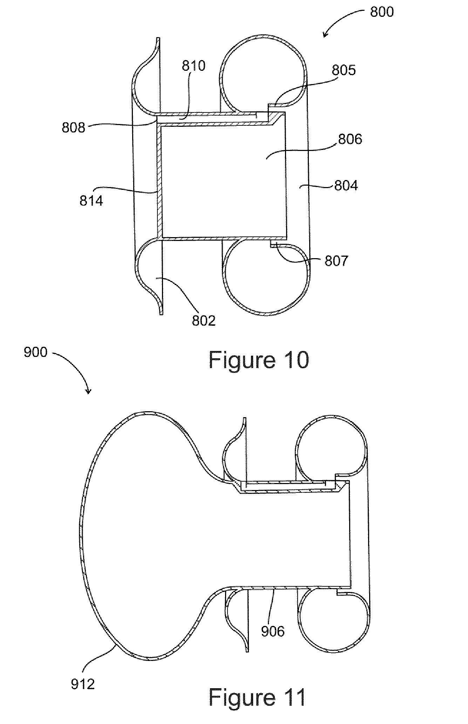

Reference is now made to Figure 10 which schematically illustrates an exemplary ostomy port **800** including an elastic stomal cover **802,** an inflatable balloon **804,** and an elastic tube **806** all integrally formed as a single component suitable for one-time use, according to some embodiments of the present invention. Ostomy port **800** includes an inflation port **808** and an inflation lumen **810** through which balloon **804** is inflated with an inflation fluid. Optionally, balloon **804** is formed with a loose end **805** attachable to a distal exterior surface **807** of elastic tube **806** during manufacture of ostomy port **800.** Optionally, a proximal end of elastic tube **806** is fitted with a non-removable cover **814** (or seal). Additionally, ostomy port **800** is removable from a stoma and disposed of when filled with waste content. Prior to removal, balloon **804** is deflated. Optionally, balloon **804** is deflated by removing the inflation fluid through inflation lumen **810** and inflation port **808.**

Reference is now made to Figure 11 which schematically illustrates an exemplary ostomy port **900** for a one-time use with a built-in ostomy bag **912** for collecting waste content and/or irrigation fluid, according to some embodiments of the present invention. Optionally, ostomy port **900** is formed as a single component similar to stomal insert **800** shown in Figure 10 having ostomy bag **912** instead of cover **814.**

Ostomy port **900** includes an elastic tube **906** to which ostomy bag **912** is attached at a proximal end. Optionally, ostomy bag **912** is furled inside elastic tube **906** prior to deployment. Optionally, ostomy bag **912** is deployed by a user pulling on the bag. Additionally or alternatively, ostomy bag **912** is deployed by an axial pressure of the waste content or flatus on the bag. Ostomy bag **912** may have any shape suitable for collecting waste content discharged through elastic tube **906.** Alternatively, ostomy bag **912** is attached to an attachment mechanism (not shown) on elastic tube **906.**

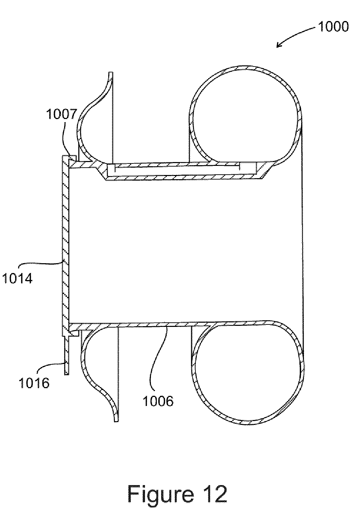

Reference is made to Figure 12 which schematically illustrates an ostomy port **1000** with a removable cap **1014** for removing waste content, according to some embodiments of the present invention. Optionally, ostomy port **1000** is formed as a single component similar to ostomy port **800** shown in Figure 10, excluding removable cap **1014** which may be separately produced from the ostomy port.

Ostomy port **1000** includes an elastic tube **1006** with a cap attachment mechanism **1007** for securing cap **1014** to the port. Removable cap **1014,** in some embodiments, has a tab **1016** for easy removal of the cap. Optionally, attachment mechanism **1007** is adapted to be fitted with a collection bag (not shown) into which waste content flows out of ostomy port **1000** following removal of cap **1014.** Optionally, the collection bag is attached to attachment mechanism **1007** following removal of cap **1014.** Optionally, cap **1014** is suitable for one-use, and is replaced with a new cap after it is being removed from ostomy port **1000.** Alternatively, cap **1014** is reusable.

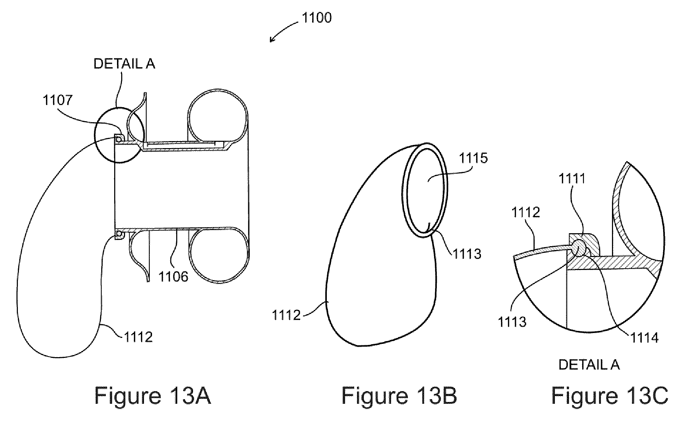

Reference is now made to Figures 13A - 13C which schematically illustrate an exemplary ostomy port **1100** including an attachment mechanism **1107** for attaching an elastic collection bag **1112,** according to some embodiments of the present invention. Optionally, ostomy port **1100** is formed as a single component similar to ostomy port **800** shown in Figure 10.

Elastic collection bag **1112** includes an opening **1115** with an annular elastic rim **1113** adapted to be inserted in a circumferential recess **1114** in attachment mechanism **1107.** Optionally, attachment mechanism **1107** includes locking mechanism **1111** for locking annular elastic rim **1113** inside circumferential recess **1114.** Additionally, attachment mechanism **1107** is adapted to support a weight of collection bag **1112** when partially full, optionally full, with waste content. Optionally, annular elastic rim **1113** includes a latex material or other type of elastomeric material. Alternatively, annular elastic rim **1113** is non-elastic and is preformed of a circumference suitable for fitting in circumferential recess **1114** and locked in place by locking mechanism **1111.**

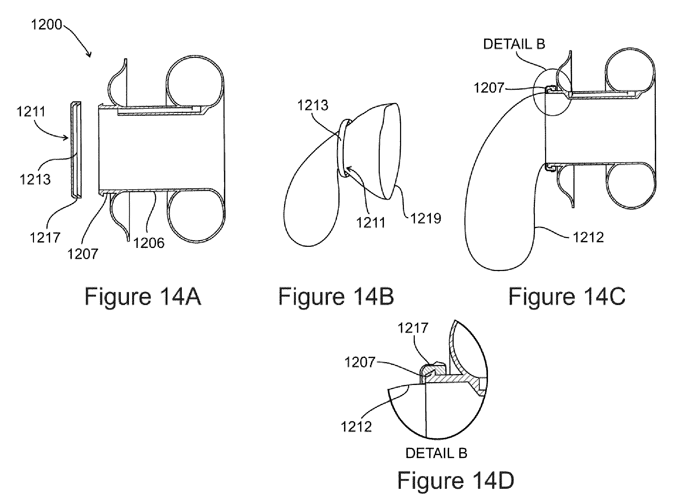

Reference is now made to Figures 14A - 14D which schematically illustrate an ostomy port **1200** including an attachment mechanism **1207** for attaching a collection bag **1212** used in domestic applications (for example, a sandwich bag, small garbage bags, and the like) to a proximal end of an elastic tube **1206,** according to some embodiments of the present invention. Optionally, ostomy port **1200** is formed as a single component similar to ostomy port **800** shown in Figure 10.

In some exemplary embodiments, attachment mechanism **1207** includes a snap-fitting arrangement for attaching a removable annular fastener **1213** having an opening **1211** through which a portion of collection bag is inserted. Optionally, annular fastener **1213** includes a latching element **1217** which snap-fits onto attachment mechanism **1207** and secures a rim portion **1219** of collection bag **1212** between the latching element and the attachment mechanism. Alternatively, other fastening arrangements known in the art may be used for removable annular fastener **1213** and attachment mechanism **1207,** for securing collection bag **1212** to ostomy port **1200.** These may include twist-locking and securing rim portion **1219** by pressing on the rim portion in an axial direction parallel to that of elastic tube **1206,** or using a spring mechanism in attachment mechanism **1207** so that the spring secures the rim portion while pressing against annular fastener **1213.**

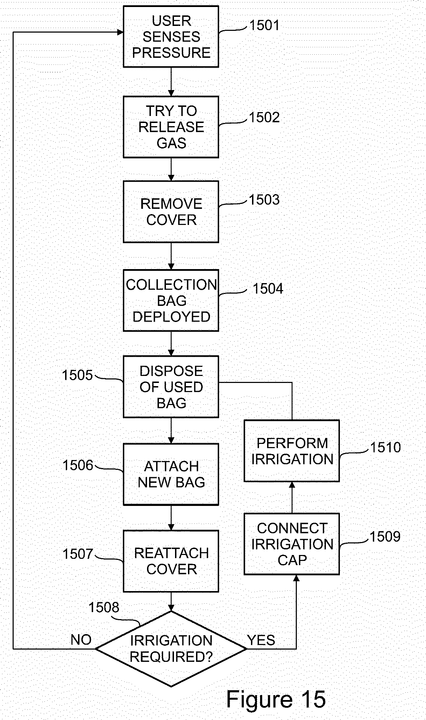

Reference is now made to Figure 15 which illustrates a flow chart of a method for using a disposable cap with an ostomy port, according to an embodiment of the present invention. The method illustrated and described herein is not intended to be limiting in any way, and an ordinary person skilled in the art may find that there are numerous other ways of implementing the method.

At **1501,** the user, during the course of the day, senses pressure from waste content which requires evacuation. Optionally, the pressure is sensed through a sensing cover attached to the cap protruding in a proximal direction. Alternatively, the pressure is electrically sensed by logic in the cap. Alternatively, the pressure is mechanically sensed by mechanical sensing components in the cap. Optionally, a visual and/or audio and/or sensible warning is provided to the user.

At **1502,** optionally, in response to the sensed pressure the user activates a gas release mechanism for releasing flatus, and then returns to stage **1501.**

At **1503,** the user removes the cover. Alternatively, the cover is ejected by the safety mechanism.

At **1504** the ostomy bag is automatically deployed by the axial pressure from the waste content pushing on the bag, allowing the waste content to flow into the bag. Alternatively, the user deploys the bag using a strap or cord that is attached to the bag, or any other method suitable for deployment of the bag.

At **1505,** the user having finished evacuation replaces the disposable ostomy bag which includes the waste content. The user releases the disposable cap from the ostomy port for replacement. The user may wipe the ostomy port with a tissue or other suitable material for removing possible residues inside port. The user then discards the used ostomy bag with the attached capsule. Optionally, the sensing cover is discarded with the capsule. Alternatively, the cover is kept for use with the replacing bag and capsule.

Optionally at **1506,** the user attaches a new cap including a new folded bag to the ostomy port.

Optionally at **1507,** the user attaches a sensing cover to the cap. Optionally, the sensing cover is that previously used. Alternatively, the cap in **1506** includes the sensing cover.

Optionally at **1508,** the user, during the course of the day, may require irrigation. If irrigation is required continue to **1509.** If irrigation is not required, go to **1501.**

Optionally at **1509,** the user detaches the regular cap and attaches a dedicated cap for irrigation. This cap may have a collection bag with higher volume, and/or supports the weight of the bag.

Optionally at **1510,** the user connects an irrigation fluid source to the ostomy port. The user removes the cover deploying the bag and opens an irrigation valve in the ostomy port. Irrigation fluid flows through the port washing out the intestine and the port. The washed out waste content flows into the bag. Optionally, the user may introduce the irrigation fluid and allow the fluid to remain inside the bowel for some time, and only then remove the cover and deploy the bag to allow bowel content flush out. The user may wipe the ostomy port with a tissue or other suitable material (optionally pre-stored in the cap) for removing possible residues inside port. Once finished irrigating, optionally go to **1505.**

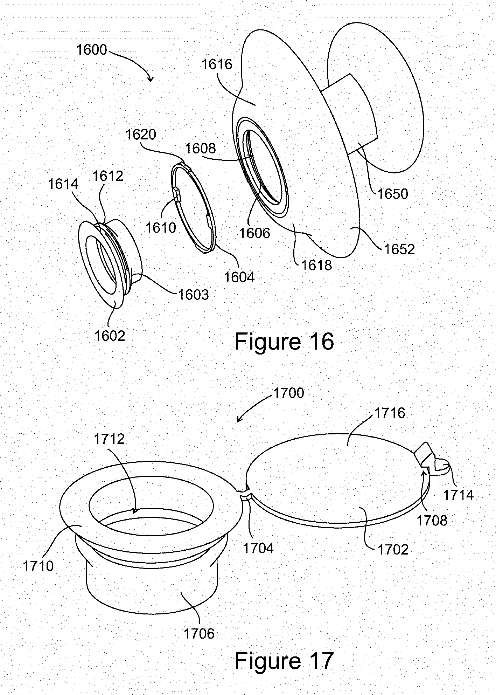

Reference is now made to Figure 16 which schematically illustrates an attachment mechanism **1600** for attaching a disposable cap **1602** with an ostomy bag inside a capsule **1603** into an ostomy port **1650,** according to some embodiments of the present invention.

An elastic ring **1604** having an oval shape is inserted (during assembly of the device at the factory) into a corresponding recess **1606** in the proximal opening of ostomy port **1650.** In order to attach cap **1602,** the user pushes the cap into proximal opening **1608.** Tabs **1610** in elastic ring **1604** are pushed apart from each other by an inclined surface **1612** in capsule **1603.** When cap **1602** is fitted into proximal opening **1608,** ring **1604** assumes its original shape and tabs **1610** fit into circumferential recess **1614** in capsule **1603.** For releasing capsule **1603,** the user presses on a top side **1616** and a bottom side **1618** of stomal cover **1652,** pressing opposing surfaces **1620** on ring **1604.** Tabs **1610** are pushed apart from each other and capsule **1603** is released.

Reference is now made to Figure 17 which schematically illustrates a quick release mechanism for removing a cover **1702** from a disposable cap **1700** for use with an ostomy port, according to some embodiments of the present invention.

Cover **1702** is formed as one component with a capsule **1706,** joined together by a strip **1704** which serves as a hinge. During assembly of cap **1700,** the assembler flips cover **1702** such that a recess **1708** locks onto a circumferential rim **1710** in capsule **1706.** Optionally, to release cover **1702** and deploy the ostomy bag inside cavity **1712,** the user presses a tab **1714** in the proximal direction to release recess **1708** from circumferential rim **1710.** Optionally, a portion of the bag (not shown) is attached (for example by bonding or welding) to an inner surface **1716** of cover **1702,** such that when the cover is opened the bag is pulled out of capsule **1706.**

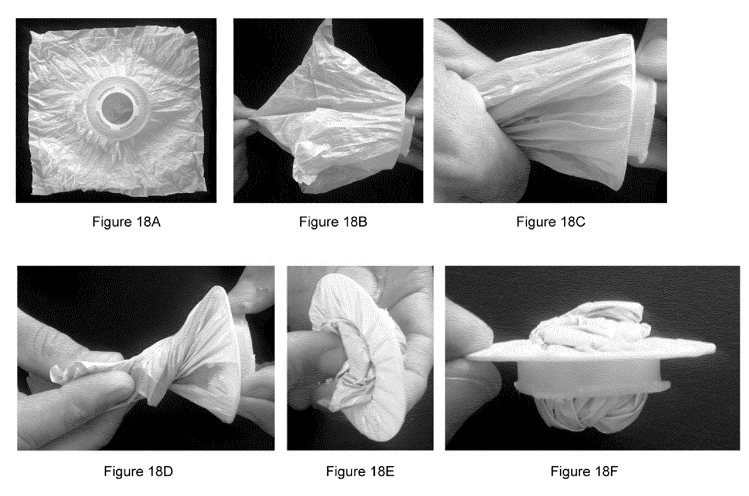

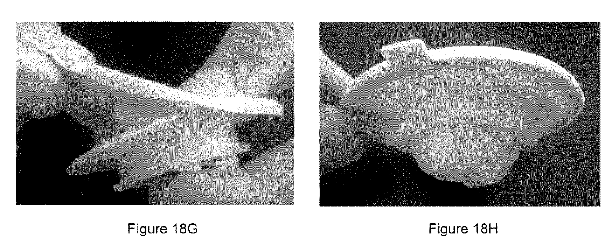

Reference is now made to Figures 18A to 18H which illustrate an exemplary method of furling an ostomy bag into a disposable cap and placing a cover on the cap, according to some embodiments of the present invention. Figure 18A illustrates the collection bag and the capsule before furling process, as viewed from the distal direction. In Figure 18B the assembler pulls a proximal portion of the collection bag apart and away from the capsule until it attains an elongated shape. In Figure 18C the assembler grasps a proximal portion of the elongated collection bag within a fist. In Figure 18D the assembler rotates said proximal portion around an axis of the capsule to 45 degrees in a clockwise direction. Alternatively, said proximal portion is rotated to 90 degrees. Alternatively, said proximal portion is rotated in the counterclockwise direction. In Figure 18E, the assembler inserts said rotated proximal portion into an internal cavity of the capsule. Figure 18F illustrates a distal view of the collection bag furled in the capsule. In Figure 18G, the assembler places a cover on the cap, securing the furled collection bag inside the capsule. Figure 18H illustrates the cap at the end of the assembly process.

The terms "comprises", "comprising", "includes", "including", "having" and their conjugates mean "including but not limited to".

The term "consisting of " means "including and limited to".

The term "consisting essentially of" means that the method or structure may include additional steps and/or parts, but only if the steps and/or parts do not materially alter the basic and novel characteristics of the claimed method or structure.

As used herein, the singular form "a", "an" and "the" include plural references unless the context clearly dictates otherwise.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the broad scope of the appended claims.

## Claims

1. A disposable cap (100) comprising:
a capsule including a coupling section adapted to seal against an ostomy port; and
a collapsed, deployable bag (102) for collecting waste content from an ostomate;
the capsule (104) includes a cavity (106, 306) accommodating said bag (102) in a collapsed state;
the cap (100) includes a sensing cover (118) attached to the capsule to close an end of the cavity (106, 306); and
the cover (118) comprises a flexible portion ;
**characterized in that**:
said flexible portion is configured to distend outward in response to an intestinal pressure (202) while the cover (118) keeps the end of the cavity (106, 306) closed.

2. The cap according to claim 1, wherein said sensing cover (118) is attached at a rim (112) located at a proximal opening (108) of the cap (100).

3. The cap according to any one of claims 1-2, wherein said sensing cover (118) is removable and replaceable from said cap (100) separately from said bag (102).

4. The cap according to any one of claims 1-3, wherein said sensing cover prevents deployment of said bag (102) while it is attached to said capsule.

5. The cap according to any one of claims 1-4, wherein said sensing cover (118) releases upon said intestinal pressure (202) reaching a predetermined pressure.

6. The cap according to claim 5, wherein said intestinal pressure (202) is in a range above 80 mmHg.

7. The cap according to any one of claims 1-6, wherein the bag is pushed out of the capsule by an axial pressure ranging between 50 mmHg and 100 mmHg.

8. The cap according to any one of claims 1-7, wherein said flexible portion is configured to distend outwards in response to a pressure of 50 mmHg or higher.

9. The cap according to any one of claims 1-8, wherein said flexible portion is fabricated from an elastomer.

10. The cap according to any one of claims 1-9, wherein said flexible portion has a thickness in the range of from 0.2-2 mm.

11. The cap according to any one of claims 1-10, wherein said intestinal pressure (202) is exerted upon said flexible portion by one or more of flatus and waste content.

12. The cap according to any one of claims 1-11, wherein said distending comprises deforming of said flexible portion by a distance within the range of 2-30 mm.

13. The cap according to any one of claims 1-12, wherein said cap (100) is shaped to extend less than 1 cm proximally from said port when sealed thereto.

14. The cap according to any one of claims 1-13, wherein said coupling section comprises a latching element (1217) configured to interlock with an attachment mechanism (1207) of said ostomy port.

15. The cap according to claim 14, wherein said latching element (1217) is comprised within an annular fastener (1213) such that said interlocking is around a rim (1219).

16. A method of deploying an ostomy bag (102) comprising:
sealing a disposable cap (100) according to any one of claims 1-15 over an ostomy;
distending a flexible portion of a cover (118) closing said cap (100), said distending being in response to an intestinal pressure;
sensing said distension; and
releasing a cover comprising said flexible portion from said cap (100).

## Patentansprüche

1. Einwegkappe (100) umfassend:
eine Kapsel mit einem Verbindungsabschnitt, der geeignet ist, gegenüber einem Ostomieanschluss abzudichten; und einem zusammengelegten,
ausfaltbaren Beutel (102) zum Sammeln von Abfall von einem Ostomiepatienten;
wobei die Kapsel (104) eine Aussparung (106, 306) umfasst, die den Beutel (102) im zusammengelegten Zustand aufnimmt;
und die Kappe eine Tastabdeckung (118) umfasst, die an der Kapsel befestigt ist, um ein Ende der Ausnehmung (106, 306) zu verschließen; und die Abdeckung (118) einen flexiblen Abschnitt umfasst,
**dadurch gekennzeichnet, dass**
der flexible Abschnitt so ausgelegt ist, dass er sich in Abhängigkeit von einem intestinalen Druck (202) nach außen dehnen kann, während die Abdeckung (118) das Ende der Ausnehmung (106, 306) verschlossen hält.

2. Kappe nach Anspruch 1, wobei die Tastabdeckung (118) an einem Rand (112) befestigt ist, der an einer proximalen Öffnung (108) der Kappe (100) angeordnet ist.

3. Kappe nach Anspruch 1 oder 2, wobei die Tastabdeckung (118) abnehmbar von der Kappe (100) und getrennt vom Beutel (102) ersetzbar ist.

4. Kappe nach einem der Ansprüche 1 bis 3, wobei die Tastabdeckung das Ausfalten des Beutels (102) verhindert, während sie an der Kapsel befestigt ist.

5. Kappe nach einem der Ansprüche 1 bis 4, wobei die Tastabdeckung (118) sich löst, wenn der intestinale Druck (202) einen vorgegebenen Wert erreicht.

6. Kappe nach Anspruch 5, wobei der intestinale Druck über 80 mmHg beträgt.

7. Kappe nach einem der Ansprüche 1 bis 6, wobei der Beutel aus der Kapsel herausgedrückt wird durch einen axialen Druck zwischen 50 mmHg und 100 mmHg.

8. Kappe nach einem der Ansprüche 1 bis 7, wobei der flexible Abschnitt so ausgelegt ist, dass er sich nach außen dehnt bei einem Druck von 50 mmHg oder mehr.

9. Kappe nach einem der Ansprüche 1 bis 8, wobei der flexible Abschnitt aus einem Elasomer hergestellt ist.

10. Kappe nach einem der Ansprüche 1 bis 9, wobei der flexible Abschnitt eine Dicke im Bereich von 0,2 bis 2 mm aufweist.

11. Kappe nach einem der Ansprüche 1 bis 10, wobei der intestinale Druck (202) auf den flexiblen Abschnitt ausgeübt wird durch Flatus und/oder Abfall.

12. Kappe nach einem der Ansprüche 1 bis 11, wobei die Ausdehnung die Verformung des flexiblen Abschnitts in einem Bereich von 2 bis 30mm umfasst.

13. Kappe nach einem der Ansprüche 1 bis 12, wobei die Kappe (100) so geformt ist, dass sie weniger als 1 cm proximal vom Ostomieanschluss hervorsteht, wenn sie damit verbunden ist.

14. Kappe nach einem der Ansprüche 1 bis 13, wobei der Verbindungsabschnitt ein Rastelement (1217) umfasst, das so ausgelegt ist, dass es mit einem Befestigungsmechanismus (1207) des Ostomieanschlusses verriegelbar ist.

15. Kappe nach Anspruch 14, wobei das Rastelement (1217) in einem ringförmigen Verschluss (1213) enthalten ist, so dass die Verriegelung um einen Rand (1219) erfolgt.

16. Verfahren zum Ausfalten eines Stomiebeutels (102) umfassend:
Verschließen eines Ostomieanschlusses mit einer Einwegkappe (100) nach einem der Ansprüche 1 bis 15;
Dehnen eines flexiblen Abschnitts einer Abdeckung (118), die die Kappe (100) schließt, wobei die Dehnung in Abhängigkeit von einem intestinalen Druck erfolgt;
Fühlen der Dehnung; und
Lösen einer Abdeckung, die den flexiblen Abschnitt der Kappe (100) umfasst.

## Revendications

1. Bouchon jetable (100) comprenant:
une capsule ayant une section de couplage conçue pour coupler étanchement à un orifice de stomie;
et une poche (102) pliée et dépliable pour collecter des matières fécales d'un patient à stomie;
la capsule (104) comprenant une cavité (106, 306) pour loger ladite poche (102) dans son état plié;
le bouchon (100) comprenant un couvercle de détection (118) fixé à la capsule pour obturer une extrémité de la cavité (106, 306); et
le couvercle comprenant une partie flexible;
**caractérisé en ce que**
ladite partie flexible est configurée pour s'étendre vers l'extérieur en fonction d'une pression intestinale (202) pendant que le couvercle (118) obture l'extrémité de la cavité (106, 306).

2. Bouchon selon la revendication 1, le couvercle de détection (118) étant fixé à un bord (112) situé à une ouverture proximale (108) dudit bouchon (100).

3. Bouchon selon l'une des revendications 1 ou 2, le couvercle de détection (118) étant amovible dudit bouchon (100) et remplaçable séparément de ladite poche (102).

4. Bouchon selon l'une quelconque des revendications 1 à 3, ledit couvercle empêchant le déploiement de ladite poche (102) pendant qu'il est fixé au bouchon.

5. Bouchon selon l'une quelconque des revendications 1 à 4, le couvercle de détection (118) se débloquant si la pression intestinale (202) dépasse une valeur déterminée.

6. Bouchon selon la revendication 5, la pression intestinale étant plus de 80 mmHg.

7. Bouchon selon l'une quelconque des revendications 1 à 6, ladite poche étant poussée hors dudit capsule par une pression axiale dans la plage entre 50 mmHg et 100 mmHg.

8. Bouchon selon l'une quelconque des revendications 1 à 7, ladite partie flexible étant configurée pour s'étendre vers l'extérieur en réponse à une pression de 50 mmHg ou plus.

9. Bouchon selon l'une quelconque des revendications 1 à 8, ladite partie flexible consistant d'un élastomère.

10. Bouchon selon l'une quelconque des revendications 1 à 9, ladite partie flexible ayant une épaisseur dans la plage de 0,2 à 2 mm.

11. Bouchon selon l'une quelconque des revendications 1 à 10, ladite pression intestinale (202) étant exercée sur ladite partie flexible par flatulences et/ou matières fécales.

12. Bouchon selon l'une quelconque des revendications 1 à 11, ladite extension comprend la déformation de ladite partie flexible de 2 à 30 mm.

13. Bouchon selon l'une quelconque des revendications 1 à 12, ledit bouchon (100) étant modelé pour s'étendre moins de 1 cm proximal dudit orifice si fixé là-dessus.

14. Bouchon selon l'une quelconque des revendications 1 à 13, ladite section de couplage comprenant un élément de verrouillage (1217) configuré pour se verrouiller sur un mécanisme de fixation (1207) dudit orifice.

15. Bouchon selon la revendication 14, ledit élément de verrouillage étant compris dans un support annulaire (1213) tel que ledit verrouillage est agencé autour d'un bord (1219).

16. Méthode de déploiement d'une poche de stomie (102) comprenant:
fixer un bouchon jetable (100) selon l'une quelconque des revendications 1 à 15 sur une stomie;
détendre une partie flexible d'un couvercle (118) obturant ledit bouchon (100), ladite détention étant in réponse à une pression intestinale;
détecter ladite détention; et
débloquer un couvercle comprenant ladite partie flexible dudit bouchon (100).
